(19) Europäisches Patentamt
European Patent Office
Office européen des brevets

(11) Publication number: 0 212 617 B1

(12) # EUROPEAN PATENT SPECIFICATION

(45) Date of publication of patent specification: 11.01.89

(51) Int. Cl.⁴: **C 07 C 57/38**, C 07 C 51/00, C 07 D233/00, C 07 D277/34, C 07 D277/36, C 07 D263/42

(21) Application number: 86111429.6

(22) Date of filing: 19.08.86

(54) **Process for producing 4-biphenylylacetic acid.**

(30) Priority: 23.08.85 JP 184084/85
23.08.85 JP 184085/85

(43) Date of publication of application: 04.03.87 Bulletin 87/10

(45) Publication of the grant of the patent: 11.01.89 Bulletin 89/02

(84) Designated contracting states: AT BE CH DE FR GB IT LI LU NL

(56) References cited:
EP-A- 0 037 479
DE-A- 2 533 397
GB-A- 1 167 192

(73) Proprietor: LEDERLE (JAPAN) LTD.
Hattori Bldg., 5th Floor 10-3 Kyobashi 1-chome
Chuo-ku Tokyo (JP)

(72) Inventor: Shimada, Osamu
26-41, Hanezawa 1-chome
Fujimi-shi Saitama-ken (JP)
Inventor: Aoyagi, Sakae
3-6-1004, Tate 2-chome
Shiki-shi Saitama-ken (JP)
Inventor: Nagase, Yunosuke
2-7, Nishi-Oizumi 3-chome
Nerima-keu Tokyo (JP)

(74) Representative: Kraus, Walter, Dr. et al
Patentanwälte Kraus, Weisert & Partner Thomas-Wimmer-Ring 15
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 212 617 B1

Jouve, 18, rue St-Denis, 75001 Paris, France

## Description

### Background of the invention

1. Field of the Invention

This invention relates to a process for producing 4-biphenylylacetic acid or its salt, and more specifically, to a simple process for producing 4-biphenylylacetic acid or its salt using 4-biphenyllylaldehyde as a starting material.

2. Description of the Prior Art

4-Biphenylylacetic acid represented by the following formula I-1 is a known compound having excellent anti-inflammatory, analgesic and antipyretic activities. A sodium salt and alkyl esters of this compound are now being widely tested as anti-inflammatory and analgesic agents for humans.

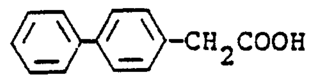

(I-1)

The following processes have previously been known typically for the production of 4-biphenylylacetic acid of formula I-1.

Process A

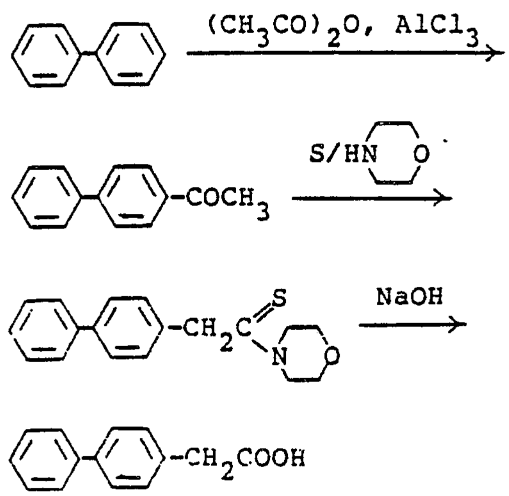


(I-1)

A process for producing 4-4-biphenylylacetic acid which comprises converting biphenyl into 4-biphenylylacetyl by the Friedel-Crafts reaction with acetic anhydride-aluminum chloride, and then subjecting 4-biphenylylacetyl to the Willgerodt reaction.

Process B

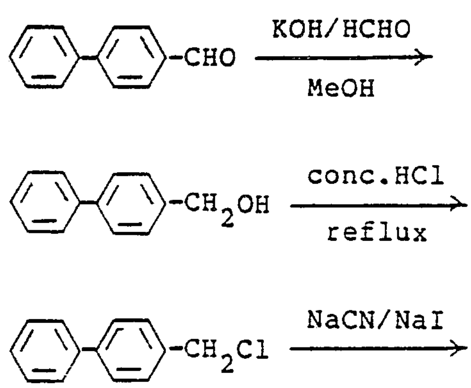

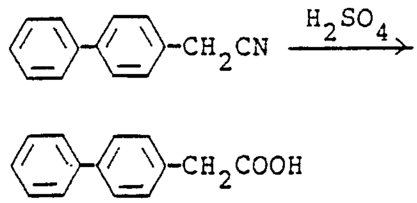


(I-1)

A process for producing 4-biphenylylacetic acid, which comprises using 4-biphenylylaldehyde as a starting material and subjecting it to a series of steps of reduction, chlorination, nitrilation and hydrolysis.

Process C

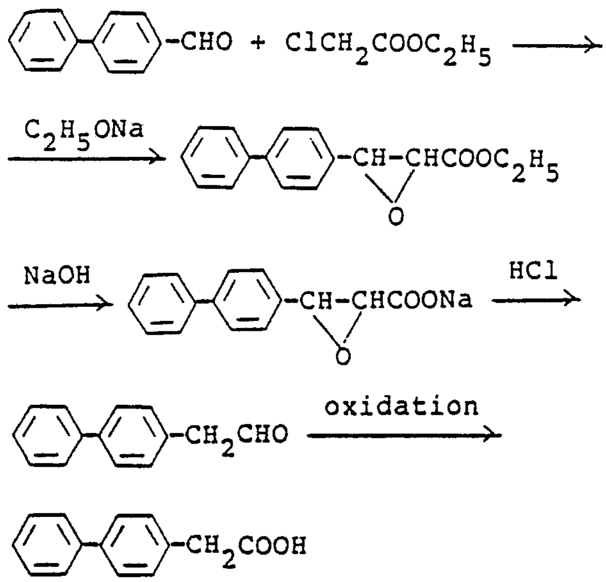


(I-1)

A process for producing 4-biphenylylacetic acid, which comprises reacting 4-biphenylylaldehyde with ethyl chloroacetate to form 4-biphenylylpropionaldehyde in which the side chain is elongated by one carbon atom, and then oxidizing the resulting compound.

According to process A, biphenyl as a starting material is available at a low cost, but long periods of time are required to perform treating operations in the steps necessary for the production of 4-biphenylylacetic acid. Furthermore, since the process goes through a sulfur compound as an intermediate, offensive odors are released and large quantities of by-products occur. Hence, a complex purifying operation is required to produce the desired 4-biphenylylacetic acid in a good purity. The yield of the final product, too, is not so good. In addition, process A has the industrial disadvantage that the entire steps cannot be performed in a single reactor (one-pot).

Process B has a number of process steps and therefore results in a decrease in yield. Furthermore, as is the case with process A, a one-pot operation cannot be employed. Moreover, since very poisonous sodium cyanide is used, process B cannot virtually be applied to industrial production of 4-biphenylylacetic acid.

According to process C, the treatment operations can be easily carried out since the reaction conditions are not so severe. But it has a number of steps, and the yield of the product in condensation between 4-biphenylylaldehyde and ethyl chloroacetate is not so high. In addition, large amounts of by-products occur, and a complex purifying step is necessary.

With the above background, the present inventors have extensively made investigations in order to establish a simple industrial process for producing 4-biphenylylacetic acid in good yields. These investigations have led to the discovery that 4-biphenylylacetic acid can be easily produced in good yields under mild conditions by using 4-biphenylylaldehyde as a starting material, reacting it with an N-acylglycine or lactam compound to form azlactone-like compound, converting the resulting compound into alpha-keto acid, etc., and decarboxylating or heat-treating alpha-keto acid, etc. ; and that since the entire steps of this process can be carried out in a single reactor (one-pot), this process is suitable for the industrial production of 4-biphenylylacetic acid.

## Summary of the invention

The present invention provides a process for producing 4-biphenylylacetic acid or its salt represented by the following formula I

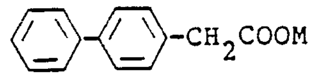

(I)

wherein M represents an alkali metal or a hydrogen atom, which comprises either (a) reacting 4-biphenylylaldehyde represented by the following formula II

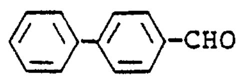

(II)

with an N-acylglycine represented by the following formula III

$$RCO—NH—CH_2COOH \quad (III)$$

wherein R represents a substituted or unsubstituted phenyl or lower alkyl group, in the presence of a base to convert 4-biphenylylaldehyde into an azlactone compound represented by the following formula V

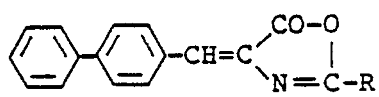

(V)

wherein R is as defined above, heating the resulting azlactone compound of formula V, and if desired, neutralizing it to form an alpha-keto acid represented by the following formula VII

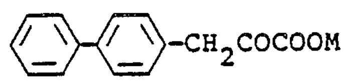

(VII)

wherein M is as defined above, and thereafter decarboxylating the alpha-keto acid ; or (b) reacting 4-biphenylylaldehyde of the following formula II

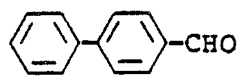

(II)

with a lactam compound represented by the following formula IV

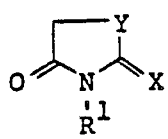

(IV)

wherein $R^1$ represents a hydrogen atom, a lower alkyl group or a carboxy-lower alkyl group, X represents an oxygen or sulfur atom, and Y represents a sulfur atom or —NH—, in the presence of a base to form a condensate represented by the following formula VI

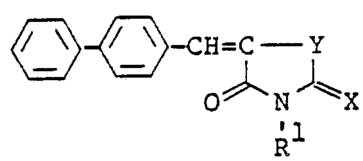

(VI)

wherein $R^1$, X and Y are as defined above, heating the resulting condensate of formula VI in the presence of an alkali base, and if desired, neutralizing it to form a derivative represented by the following formula VIII

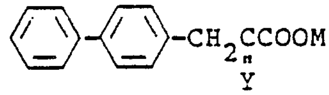

(VIII)

wherein M and Y are as defined above, and treating the derivative of formula VIII with a peroxide in the presence of an aqueous base.

More specifically, as a first variant, the present invention provides as a first variant a process which

comprises reacting 4-biphenylylaldehyde of the following formula II

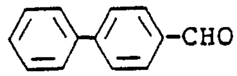

(II)

with an N-acylglycine represented by the following formula III

$$RCO—NH—CH_2COOH \quad (III)$$

wherein R represents an unsubstituted or substituted phenyl or lower alkyl group, in the presence of a base to convert 4-biphenylylaldehyde into an azlactone compound represented by the following formula V

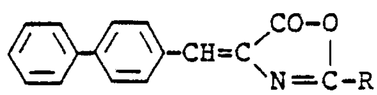

(V)

wherein R is as defined above, heating the resulting azlactone compound of formula V in the presence of an alkali metal base and if desired, neutralizing it to form an alpha-keto acid represented by the following formula VII

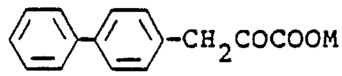

(VII)

wherein M represents an alkali metal or a hydrogen atom, and thereafter decarboxylating the alpha-keto acid.

As a second variant, the present invention provides a process which comprises reacting 4-biphenylylaldehyde of formula II with a lactam compound represented by the following formula IV

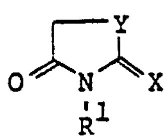

(IV)

wherein $R^1$ represents a hydrogen atom, a lower alkyl group or a carboxy-lower alkyl group, X represents an oxygen or sulfur atom, and Y represents a sulfur atom or —NH—, in the presence of a base to form a condensate represented by the following formula VI

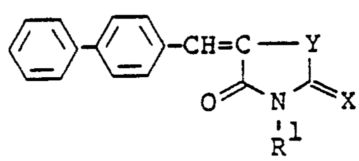

(VI)

wherein $R^1$, X and Y are as defined above, heating the condensate of formula VI in the presence of an alkali base and if desired, neutralizing it to form a derivative represented by the following formula VIII

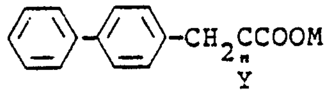

(VIII)

wherein Y is as defined above, and M represents an alkali metal or a hydrogen atom, and thereafter treating the resulting derivative in an aqueous solvent.

The process variants of this invention can be carried out as a one-pot process by which all the steps are carried out in the same reactor without isolating the compound of formula V or VI resulting from reaction of 4-biphenylylaldehyde with the compound of formula III or IV and the compound of formula VII or VIII subsequently obtained. According to a particularly preferred embodiment, the process of this invention can be carried out by heat-treating 4-biphenylylaldehyde of formula II and the compound of formula III or IV in the presence of a base and subsequently decarboxylating the product or treating it with a peroxide in the presence of an aqueous base in the same reactor. This embodiment is particularly preferred as an industrial simple process.

## Detailed description of the invention

In the process of this invention, 4-biphenylylaldehyde of formula II and the compound of formula III or IV are used as starting materials. The process is schematically shown below by chemical formulae.

First process variant

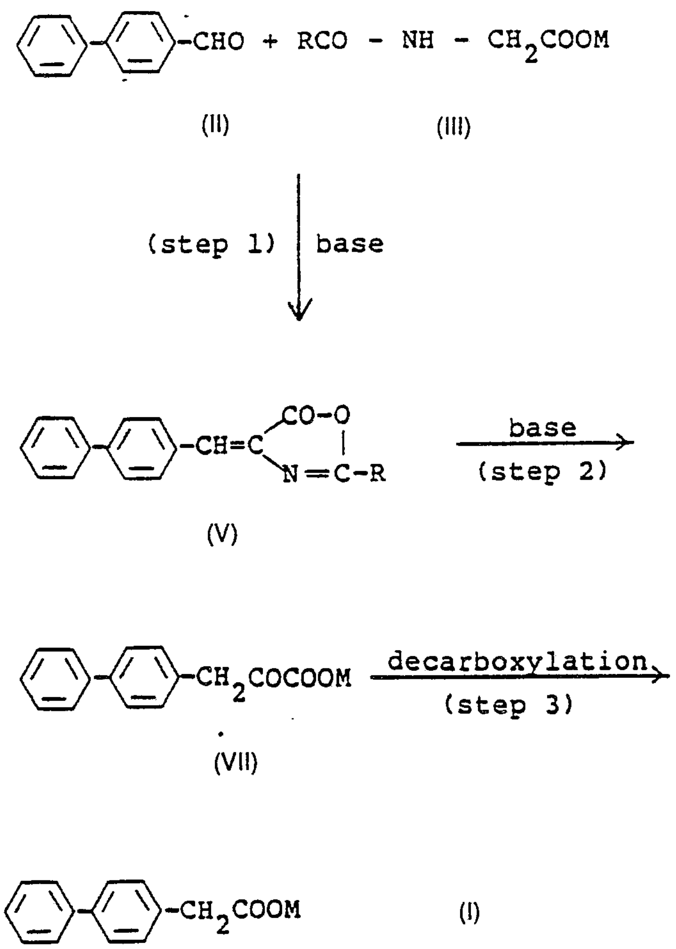


(II) (III)

(V)

(VII)

(I)

(In the above chemical formulae, R and M are as defined hereinabove.)

Second process variant

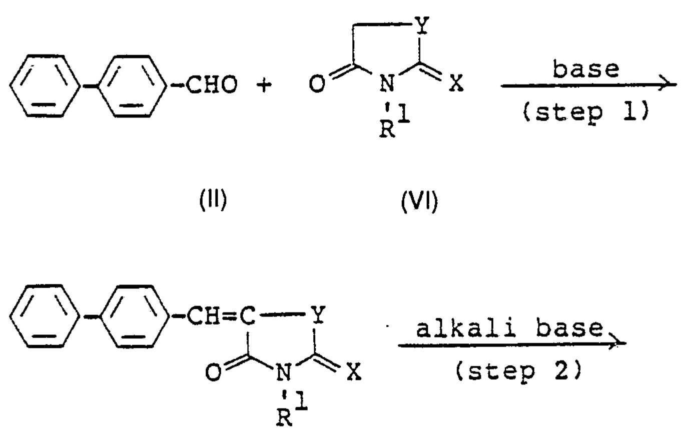


(II) (VI)

(VI)

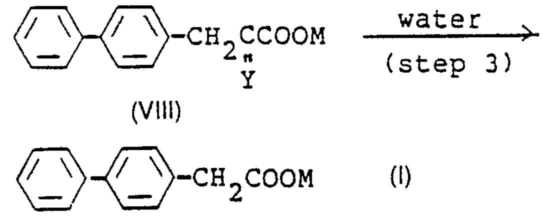


(VIII)

(I)

(In the above chemical formulae, $R^1$, X, Y and M are as defined hereinabove.)

In the process of this invention, steps 1, 2 and 3 in each of the above process variants may be carried out individually, or in the same reactor as a one-pot process.

The term « lower », used throughout the present specification and claims to qualify a group or a compound, means that the group or compound so qualified contains not more than 6, preferably not more than 4, carbon atoms.

The « lower alkyl group », as used in the present specification and claims, denotes a linear or branched alkyl group having 1 to 6 carbon atoms. Examples include methyl, ethyl, n-propyl, iso-propyl, n-butyl, iso-butyl, sec-butyl, tert-butyl, n-pentyl, iso-pentyl, neo-pentyl and n-hexyl groups.

The substituent R in the N-acylglycine represented by formula III is an unsubstituted or substituted phenyl or lower alkyl group. Examples include a phenyl group ; a phenyl group substituted by 1 to 3, preferably 1 or 2, substituents selected from lower alkyl groups, lower alkoxy groups and halogen atoms, such as p-tolyl, 4-chlorophenyl, 4-methoxyphenyl and 3,4-dimethoxyphenyl groups ; and linear or branched alkyl groups having 1 to 6 carbon atoms such as methyl, ethyl, n-propyl, iso-propyl, n-butyl, sec-butyl, t-butyl, n-pentyl, iso-pentyl, neo-pentyl and n-hexyl groups.

The « carboxy-lower alkyl group » is a lower alkyl group substituted by the carboxyl group, such as carboxymethyl, alpha-carboxyethyl, alpha-carboxypropyl and beta-carboxyethyl groups. Those having the carboxyl group at the alpha-position are preferred.

Specific examples of the N-acylglycine of formula III used in the first process variant are N-benzoylglycine, N-(p-toluoyl)glycine, N-anisoylglycine, N-acetylglycine, N-propionylglycine and N-(n-butyryl) glycine. Of these, N-benzoylglycine is preferred because it is easily available at a low cost.

Examples of the lactam compound of formula IV used in the second process variant typically include 2-thioxo-4-thiazolidinone (rhodanine), N-methyl-2-thioxo-4-thiazolidinone, 2,4-imidazolidinone (hydantoin) and N-carboxymethyl-2-thioxo-4-thiazolidinone.

The first and second process variants in accordance with this invention will be described in more detail with reference to the individual steps schematically shown above. (In the case of the one-pot process, the conditions of steps 1 to 3 may be properly combined and the process can be performed in a single reactor. This will also be described hereinafter.)

Step 1

In this step, 4-biphenylylaldehyde of formula II is reacted with the N-acylglycine of formula III or the lactam compound of formula IV in the presence of a base to form the azlactone compound of formula V or the condensate of formula VI.

The reaction is carried out in the presence of a base with or without a solvent. Examples of the base that can be used in this reaction are ammonia ; organic tertiary amines such as trimethylamine, triethylamine, benzyldimethylamine, benzyldiethylamine, N-methylmorpholine, N-ethylpiperidine and N,N-diisopropylethylamine ; aromatic amines such as pyridine and 2-pyrroline ; metal salts of organic acids such as sodium formate, sodium acetate and potassium acetate ; and inorganic bases, for example carbonates and bicarbonates of alkali metals and alkaline earth metals such as lithium carbonate, potassium carbonate, sodium bicarbonate, potassium bicarbonate, lithium carbonate, calcium carbonate and barium carbonate. Of these, sodium acetate, potassium acetate, and triethylamine are preferred. Sodium acetate is especially preferred. Since 4-biphenylylaldehyde of formula II is a liquid and has a low boiling point, it can concurrently serve as a solvent and therefore, the reaction may be carried out in the absence of solvent. Alternatively, the reaction may be carried out in the presence of a solvent which does not directly participate in the reaction.

Specific examples of such a solvent include polar aprotic solvents such as acetonitrile, dimethylformamide, dimethylacetamide and dimethyl sulfoxide ; ethers such as tetrahydrofuran and dioxane ; inert alkyl halides such as dichloromethane and chloroform ; aromatic solvents such as benzene, toluene, xylene, chlorobenzene, dichlorobenzene and nitrobenzene ; and organic acids and organic acid anhydrides such as acetic acid and acetic anhydride. Of these, toluene, chlorobenzene and dioxane are preferred.

In this step, it is convenient to perform the reaction using generally 1 to 3 moles, preferably 1 to 1.5 moles of the N-acylglycine of formula III or the lactam compound of formula IV per mole of 4-biphenylylaldehyde of formula II in the presence of generally 1 to 5 moles, preferably 1 to 2 moles, of the base and about 1 to 10 moles, preferably 2 to 5 moles, of acetic anhydride.

The reaction temperature and time are not restricted in a generally manner because they vary

depending upon the type of the base used and the presence or absence of the solvent. Generally, in the absence of solvent, the temperature is about 0 to 150 °C, preferably about 50 to about 100 °C, and the time is about 0.1 to about 5 hours, preferably about 0.5 to about 1.5 hours. In the case of using a solvent, the reaction is suitably carried out at a temperature near the boiling point of the used solvent for about 0.1 to about 12 hours, preferably about 0.5 to about 5 hours.

In the first step, the azlactone compound of formula V or the condensate of formula VI can be obtained mainly as crystals in a yield of about 90 % or in a quantitative yield. This compound may be used in the next step 2 without purification.

Step 2

In this step, the azlactone of formula V or the condensate of formula VI obtained in step 1 is heated in the presence of a base to convert it into the compound of formula VII or VIII.

Examples of the base used in this step are hydroxides of alkali metals or alkaline earth metals such as sodium hydroxide, potassium hydroxide, lithium hydroxide, calcium hydroxide and magnesium hydroxide, and the same carbonates or bicarbonates of such metals as described with regard to step 1. The amount of the base can generally be 1 to 5 moles, preferably 1 to 2 moles, per mole of the compound of formula V or VI.

In this reaction, the compound of formula V or IV and the base are heated usually in the presence of a solvent. The solvent used at this time may, for example, be watter alone or a mixture of water with any of the various organic solvents illustrated above with regard to step 1.

The heating temperature is generally about 50 to about 150 °C, preferably about 100 to about 120 °C. When the aforesaid mixture of water and an organic solvent is used, the heating may conveniently be carried out at a temperature near the boiling point of the organic solvent. At the above temperature, the reaction comes to completion usually in about 0.5 to about 15 hours, preferably about 1 to about 10 hours. As a result, the desired compound of formula VII or VIII can be obtained in a nearly quantitative yield.

In this case, the compound of formula VII or VIII is obtained in the form of a salt corresponding to the base used. When, for example, sodium hydroxide is used as the base, the compound is obtained as a sodium salt (M = Na). The salt of the compound may be converted to a free compound (M = H) by neutralizing it with an acid in a customary manner.

Step 3

In this step, the alpha-keto acid of formula VII obtained in step 2 is decarboxylated or the compound of formula VIII is treated with a peroxide in an aqueous base, thereby to obtain 4-biphenylylacetic acid of formula I. This step can be carried out by methods known per se. Decarboxylation of the compound of formula VII and treatment of the compound of formula VIII are carried out in the same manner. For example, it is carried out by treating the compound of formula VII or VIII with an alkali metal hydroxide such as sodium hydroxide or potassium hydroxide and a peroxide such as hydrogen peroxide in the presence of a suitable amount of water.

The above reaction can be carried out by treating the compound of formula VII or VIII with usually 1 to 10 moles, preferably 1 to 3 moles, per mole of the compound of formula VII or VIII, of an alkali metal hydroxide such as sodium hydroxide and generally 1 to 10 moles, preferably 1 to 5 moles, per mole of the compound of formula VII or VIII, of a peroxide such as hydrogen peroxide at a temperature in the range of about 0 to about 100 °C, preferably about 10 to about 40 °C, for a period of about 1 to about 20 hours, preferably about 5 to about 10 hours. As a result, the desired 4-biphenylylacetic acid or its salt represented by formula I can be obtained in good yields.

When in this step 4-biphenylylacetic acid of formula I is obtained in the form of a salt such as a sodium salt, it may as required be neutralized to convert it to a free acid, and the desired 4-biphenylylacetic acid may be obtained in pure form by applying known isolating and purifying means such as extraction, chromatography and recrystallization to the free acid.

As stated above, in steps 1, 2 and 3 in accordance with this invention, the final products in these steps can be obtained in good yields by a simple means such as heating. The present invention is particularly characterized in that these steps 1, 2 and 3 can be carried out in a single reactor (one-pot process). This one-pot process will be described in detail below.

One-pot process

The one-pot process may be carried out by properly selecting those conditions which can be practiced in a single reactor from the conditions in steps 1, 2 and 3 described hereinabove. Specifically, it is preferably carried out as follows : In the following, the one-pot process will be described with regard to the first process variant, but the second process variant may also be carried out by a one-pot technique in the same way.

In the first step, 1 mole of 4-biphenylylaldehyde represented by formula II is reacted with 1 to 3 moles, preferably 1 to 1.5 moles, of the N-acylglycine of formula III in the presence of sodium acetate, for

example, as a base in the absence of a solvent or in a solvent such as toluene. In this case, sodium acetate and toluene as the base and solvent are selected because they can be directly used in the conversion reaction to the alpha-keto acid in the second step.

Thereafter, as the second step, the reaction mixture in the first step is directly heated to a temperature in the range of about 80 to about 150 °C at the end of the treatment in the first step. Alternatively, the solvent is evaporated, and if desired, a base for example a sodium hydroxide solution is further added, and the resulting mixture is heated to a temperature in the aforesaid range.

In the treatment of the second step, the azlactone compound of formula V formed in the reaction system by the treatment of the first step is converted to the alpha-keto acid of formula VII quantitatively.

Thereafter, as the third step, a desired amount of a peroxide, for example, about 2 to 10 moles, per 4-biphenylylaldehyde used in the first step, of hydrogen peroxide is added to the reaction mixture as obtained in the second step, and the mixture was stirred at a temperature of about 10 to about 40 °C for about 5 to about 10 hours.

In the third step, the alpha-keto acid of formula VII is converted to 4-biphenylylacetic acid or its salt of formula I which is the final product of this invention.

After the treatments in the first to third steps have been carried out in the same reactor, the contents are optionally neutralized, and then subjected to a suitable combination of extraction, distillation and recrystallization to give 4-biphenylylacetic acid as a pure product.

The yield of the final product in the one-pot process is as high as 70 to 80 % based on the phenylylaldehyde used in the first step.

Hence, the process of this invention is simpler in operation and higher in yeild than the conventional processes A to C described above, and is especially advantageous for industrial production.

The steps of the process of this invention is relatively small, and the treatments in the individual steps are, for example, heating and strirring which are simple. Moreover, the reaction conditions are not severe. Accordingly, the productivity of the process of this invention is very good as an industrial process.

## Examples

The following Examples illustrate the process of this invention both in a stepwise mode and in a one-pot mode. It should be understood, however, that the invention is never limited to these examples alone.

### Example 1

To a solution of 4-biphenylylaldehyde (5g, 0.027 mol) in acetic anhydride (8 ml) were added N-benzoylglycine (5.47 g, 0.034 mol) and sodium acetate (2.3 g). The mixture was stirred at 40-50 °C for 2 hours, then poured into hot ethanol, and cooled. The precipitated crystals were collected by filtration to give 8.3 g (94 % yield) of 2-phenyl-4-(4′-biphenylylmethylidene)-5-oxazolone as yellow crystals. Furthermore, the filtrate was concentrated to give an additional 0.4 g (5 % yield) of the crystals. (Total yield : 99 %.)

mp : 187-188 °C
IR spectrum (KBr) : 1 790 $cm^{-1}$
UV spectrum : $\lambda$ max 380 nm

### Example 2

A mixture of 2-phenyl-4-(4′-biphenylylmethylidene)-5-oxazolone (3 g, 9.2 mmol) prepared in Example 1 and 20 % sodium hydroxide solution (15 ml) was refluxed for 2 hours. After cooling, the mixture was washed with benzene, acidified with hydrochloric acid, and extracted with ethyl acetate. The combined extracts were washed with water and brine, and dried over anhydrous sodium sulfate. Evaporation of the solvent in vacuo gave 2.0 g (90 % yield) of 4-biphenylylpyruvic acid as a crystalline solid, which was recrystallized from ethyl acetate.

mp : ca. 205 °C (sublimed)
IR spectrum (KBr) : 1 660 $cm^{-1}$

### Example 3

To 2 g (8.3 mmol) of 4-biphenylylpyruvic acid prepared in Example 2 were added 0.5 % sodium hydroxide solution (15 ml) and 30 % hydrogen peroxide (4 ml). After being stirred at 20-30 °C for 4 hours, the mixture was washed with toluene, acidified with hydrochloric acid, and extracted with ethyl acetate. The combined extracts were washed with water and brine, and dried over anhydrous sodium sulfate. Evaporation of the solvent in vacuo gave 1.6 g (91 %) of 4-biphenylylacetic acid as a crystalline solid, which was recrystallized from methyl ethyl ketone.

mp : 163-164 °C
IR spectrum (KBr) : 1 688 $cm^{-1}$

Example 4

4-Biphenylylaldehyde (4 g, 0.022 mol) was dissolved in acetic anhydride (4 ml) and N-benzoylglycine (4.33 g, 0,024 mol), sodium acetate (1.84 g) and toluene (28 ml) were added to the solution. After refluxing for 2 hours, 20 % sodium hydroxide solution (65 ml) was added to the reaction mixture. The mixture was refluxed for 2 hours, washed with benzene and extracted with ethyl acetate. The extract was washed with water and then with brine, dried over anhydrous sodium sulfate an concentrated in vacuo to give 5.2 g (99 % yield) of 4-biphenylylpyruvic acid as a crystalline solid.

IR spectrum (KBr) : 1 660 $cm^{-1}$

Example 5

To 3 g (9.2 mmol) of 2-phenyl-4-(4'-biphenylmethylidene-5-oxazolone obtained in Example 1 was added 15 ml of sodium hydroxide solution and the mixture was refluxed for 2 hours. After being cooled to about 10 °C, 40 % sodium hydroxide solution (4 ml) and 30 % hydrogen peroxide were added to the solution and the mixture stirred at room temperature for 6.5 hours. The resulting solution was acidified with hydrochloric acid and extracted with benzene. The extract was washed with water and then brine, dried over anhydrous sodium sulfate and concentrated in vacuo to give 1.6 g (81 % yield) of 4-biphenylylacetic acid. The melting point and spectral data of the crystals obtained were identical with those of the product prepared in Example 3.

Example 6

4-Biphenylylaldehyde (5 g, 0.027 mol) was dissolved in 8 ml of acetic anhydride, and N-benzoylglycine (5.47 g, 0.034 mol), sodium acetate (2.3 g) and toluene (35 ml) were added to the solution. The mixture was refluxed for 2.5 hours. Then 40 % sodium hydroxide solution was added, and the mixture refluxed for 2 hours. After cooling, 30 % hydrogen peroxide (12 ml) was added and the reaction mixture was stirred at room temperature for 5 hours. The solution was washed with cold toluene and then acidified with hydrochloric acid. The resulting crystals were collected by filtration to give 4.9 g (84 % yield) of 4-biphenylylacetic acid. The melting point and spectral data of the crystals obtained were identical with those of the product prepared in Example 3.

Example 7

4-Biphenylylaldehyde (1 kg) was dissolved in 1.5 l of acetic anhydride, and N-benzoylglycine (1.09 kg), sodium acetate (0.46 kg) and toluene (5 l) were added to the solution. The mixture was refluxed for 2.5 hours. Then 40 % sodium hydroxide solution was added, and the mixture refluxed for 4 hours. After cooling, 30 % hydrogen peroxide was added and the reaction mixture was stirred at room temperature for 6 hours. The solution was washed with cold toluene and then acidified with hydrochloric acid. The resulting crude crystals were collected by filtration and recrystallized from methyl ethyl ketone to give 0.86 kg (74 % yield) of 4-biphenylylacetic acid.

Example 8

4-Biphenylylaldehyde (3 g, 0.016 mol) was dissolved in acetic anhydride (4.8 ml), and 2-thioxo-4-thiazolidine (2.4 g, 0.018 mol), sodium acetate (1.4 g) and toluene (20 ml) were added to the solution. The mixture was refluxed for 2 hours, then extracted with ethyl acetate, washed with water, and dried. Evaporation of the solvent in vacuo gave 4.6 g (94 % yield) of 5-(4'-biphenylmethylidene)-2-thioxo-4-thiazolidinone as a yellow crystalline solid, which was recrystallized from ethyl acetate.

mp : 243-244 °C

IR spectrum (KBr) : 1 700-1 710 $cm^{-1}$

UV spectrum : $\lambda$ max 387 nm

Example 9

4-Biphenylylaldehyde (5 g, 0.027 mol) was dissolved in acetic anhydride (8 ml), and N-methyl-2-thoxo-4-thiazolidinone (4.48 g, 0.03 mol) and sodium acetate (2.3 g) were added to the solution. The mixture was heated at 80-90°C for 2.5 hours and then cooled, and benzene and water were added. The benzene layer was separated, washed with water, and dried over anhydrous sodium sulfate. The benzene solution was concentrated in vacuo to give 8.1 g (quantitative yield) of N-methyl-5-(4'-biphenylylmethylidene)-2-thioxo-4-thiazolidinone as a crystalline solid, which was recrystallized from ethyl acetate.

mp : 181-182 °C

IR spectrum (KBr) : 1 710 $cm^{-1}$

UV spectrum : $\lambda$ max 387 nm

Example 10

4-Biphenylylaldehyde (3 g, 0.016 mol) was dissolved in acetic anhydride (4.8 ml), and N-carboxymethyl-2-thioxo-4-thiazolidinone (3.2 g, 0.017 mol) and sodium acetate (1.38 g) were added to the solution. The mixture was heated at 90 °C for 1 hour. Water was added, and the mixture extracted with ethyl acetate. The combined extracts were washed with water, dried over anhydrous sodium sulfate, and concentrated in vacuo to give 5.56 g (95 % yield) of N-carboxymethyl-5-(4'-biphenylylmethylidene)-2-thioxo-4-thiazolidinone, which was recrystallized using ethyl acetate.

mp : 256-257 °C (decomposed)
IR spectrum (KBr) : 1 740 and 1 715 $cm^{-1}$
UV spectrum : λ max 388 nm

Example 11

4-Biphenylylaldehyde (3 g, 0.016 mol) was dissolved in acetic anhydride (0.5 ml), and glacial acetic acid (2.9 ml) and 2,4-imidazolidinedione (2.48 g, 0.025 mol), sodium acetate (1.38 g) and toluene (2 ml) were added to the solution. The mixture was refluxed for 9 hours. After cooling, water was added to the reaction mixture and the mixture extracted with benzene. The combined extracts were washed with water, dried over sodium sulfate, and concentrated in vacuo to give 4.0 g (92 % yield) of 5-(4'-biphenylylmethylidene)-2,4-imidazolidinedione as crystalline solid, which was recrystallized from ethyl acetate.

mp : 240 °C (decomposed)
IR spectrum (KBr) : 1 778, 1 718 and 1 658 $cm^{-1}$
UV spectrum : λ max 333 nm

Example 12

To 3 g (0.011 mol) of 5-(4'-biphenylylmethylidene)-2,4-imidazolidinedione prepared in Example 11 was added 20 % sodium hydroxide solution (10 ml) and the mixture was refluxed for 4 hours. After cooling, the reaction mixture was washed with benzene, acidified with hydrochloric acid, and extracted with ethyl acetate. The combined extracts were washed with water and brine, then dried over sodium sulfate, and concentrated to give 1.3 g (48 % yield) of 4-biphenylpyruvic acid as a crystalline solid, which was recrystallized from ethyl acetate.

mp : ca. 205 °C (sublimed)
IR spectrum (KBr) : 1 660 $cm^{-1}$

Example 13

To 2 g (8.3 mmol) of 4-biphenylylpyruvic acid prepared in Example 12 were added 0.5 % sodium hydroxide solution (15 ml) and 30 % hydrogen peroxide (4 ml). The mixture was stirred at 25-30°C for 4 hours, then washed with toluene, acidified with hydrochloric acid, and extracted with ethyl acetate. The combined extracts were washed with water and brine, and dried over sodium sulfate. Evaporation of the solvent gave 1.5 g (85 % yield) of 4-biphenylylacetic acid as a crystalline solid, which was recrystallized from methyl ethyl ketone.

mp ; 163-164 °C
IR spectrum (KBr) : 1 688 $cm^{-1}$

Example 14

A mixture of 5-(4'-biphenylylmethylidene)-2-thioxo-4-thiazolidinone (3 g, 0.01 mol) prepared in Example 8 and 20 % sodium hydroxide solution (25 ml) was refluxed for 2 hours, and 30 % hydrogen peroxide (10 ml) at 5-10 °C was added to the mixture. After being stirred at room temperature for 5 hours, the solution was washed with toluene, acidified with hydrochloric acid, and then extracted with ethyl acetate. The combined extracts were washed with water and brine, dried over anhydrous sodium sulfate, and concentrated in vacuo to give 1.1 g (51 % yield) of 4-biphenylylacetic acid as crystals. The melting point and spectral data of the crystals obtained were identical with those of the product in Example 3.

Example 15

A mixture of N-methyl-5-(4'-biphenylylmethylidene)-2-thioxo-4-thiazolidinone (2 g, 6.8 mmol) prepared in Example 9 and 20 % sodium hydroxide solution was refluxed for 2 hours. After cooling, 30 % hydrogen peroxide (5 ml) was added. The mixture was stirred at room temperature for 5 hours, washed with toluene, acidified with hydrochloric acid, and then extracted with benzene. The combined extracts were washed with water and brine, dried over anhydrous sodium sulfate, and concentrated in vacuo to give 1.1 g (76 % yield) of 4-biphenylylacetic acid as crystals. The melting point and spectral data of the

crystals were identical with those of the product in Example 3.

Example 16

4-Biphenylylaldehyde (3 g, 0.016 mol) was dissolved in acetic anhydride (4.8 ml) and 2-thioxo-4-thiazolidinone (2.4 g, 0.018 mol), sodium acetate (1.4 g) and toluene (20 ml) were added to the solution. The mixture was refluxed for 2 hours, and 40 % sodium hydroxide solution was added. The mixture was refluxed for additional 2 hours. The reaction mixture was then allowed to cool, and 30 % hydrogen peroxide was added. The mixture was stirred at room temperature for 5 hours, washed with toluene, acidified with hydrochloric acid, and extracted with ethyl acetate. The combined extracts were washed with water and brine, dried over sodium sulfate, and concentrated in vacuo to give 1.4 g (40 % yield) of 4-biphenylylacetic acid. The melting point and spectral data of the crystals obtained were identical with those of the product in Example 3.

Example 17

4-Biphenylylaldehyde (3 g, 0.016 mol) was dissolved in acetic anhydride (0.5 ml) and glacial acetic acid (29 ml). To the solution were added sodium acetate (1.4 g), 2,4-imidazolidinedione (2.48 g, 0.025 mol) and toluene (2 ml), and the mixture was refluxed for 19 hours. After addition of 20 % sodium hydroxide solution (16 ml), the mixture was refluxed for 2 hours. After cooling, 30 % hydrogen peroxide was added to the mixture, and the whole mixture was stirred at room temperature for further 5 hours. The reaction mixture was washed with toluene, acidified with hydrochloric acid, and extracted with ethyl acetate. The combined extracts were washed with water and brine, and dried over anhydrous sodium sulfate. Evaporation of the solvent gave a crystalline solid, which was recrystallized to give 1.5 g (43 % yield) of 4-biphenylylacetic acid. The melting point and spectral data of the crystals obtained were identical with those of the product in Example 3.

Example 18

A mixture of 4-biphenylylaldehyde (105 g, 0.576 mol), N-benzoylglycine (114.8 g, 0.641 mol), sodium acetate (48.3 g), acetic anhydride (168 ml) and toluene (700 ml) was heated at an internal temperature of 80-90 °C. After 4 hours, the mixture was poured into stirred cold ethanol and the resulting, yellow crystals were collected by filtration to give 166.5 g (89 % yield) of 2-phenyl-4-(4'-biphenylmethylidene)-5-oxazolone.

Example 19

A mixture of 2-phenyl-4-(4'-biphenylylmethylidene-5-oxazolone (150 g, 0.461 mol) prepared in Example 18, potassium hydroxide (207.3 g) and water (1.5 l) was refluxed with stirring for 4 hours and then cooled to 10 °C. To this mixture was added 161.6 ml of 30 % hydrogen peroxide and the whole mixture was stirred at room temperature. After 4 hours, the reaction mixture was acidified with 10 % hydrochloric acid solution. The precipitated crystals were collected by filtration and recrystallized from methyl ethyl ketone to give 78.3 g (80 % yield) of 4-biphenylylacetic acid, identical with a sample prepared in Example 3.

## Claims

1. A process for producing 4-biphenylylacetic acid or its salt represented by the following formula I

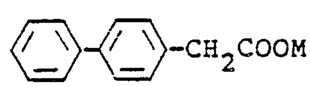

(I)

wherein M represents an alkali metal or a hydrogen atom, which comprises either

(a) reacting 4-biphenylylaldehyde represented by the following formula II

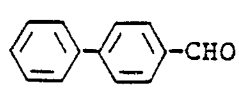

(II)

with an N-acylglycine represented by the following formula III

$$RCO—NH—CH_2COOH \quad (III)$$

wherein R represents a substituted or unsubstituted phenyl or lower alkyl group, in the presence of a base to convert 4-biphenylylaldehyde into an azlactone compound represented by the following formula V

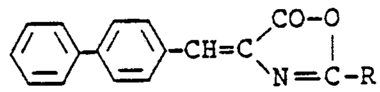

(V)

wherein R is as defined above, heating the resulting azlactone compound of formula V, and if desired, neutralizing it to form an alpha-keto acid represented by the following formula VII

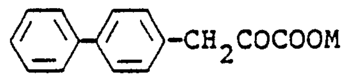

(VII)

wherein M is as defined above, and thereafter decarboxylating the alpha-keto acid ; or

(b) reacting 4-biphenylylaldehyde of the following formula II

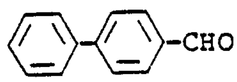

(II)

with a lactam compound represented by the following formula IV

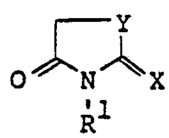

(IV)

wherein $R^1$ represents a hydrogen atom, a lower alkyl group or a carboxy-lower alkyl group, X represents an oxygen or sulfur atom, and Y represents a sulfur atom or —NH—, in the presence of a base to form a condensate represented by the following formula VI

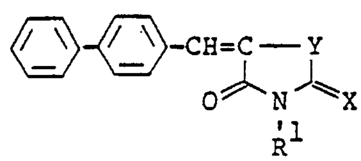

(VI)

wherein $R^1$, X and Y are as defined above, heating the resulting condensate of formula VI in the presence of an alkali base, and if desired, neutralizing it to form a derivative represented by the following formula VIII

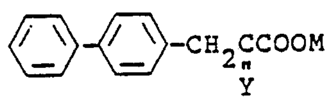

(VIII)

wherein M and Y are as defined above, and treating the derivative of formula VIII with a peroxide in the presence of an aqueous base.

2. The process of claim 1 wherein the process variant (a) is carried out in a single reactor without isolating the azlactone compound of formula V and the alpha-keto compound of formula VII.

3. The process of claim 2 wherein in a single reactor, the 4-biphenylylaldehyde of formula II and the N-acylglycine of formula III are heat-treated in the presence of a base, followed by decarboxylation.

4. The process of claim 1 wherein the decarboxylation is carried out by treatment with a peroxide under basic conditions.

5. An azlactone compound which is represented by the following formula V

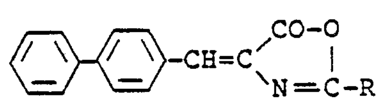

(V)

wherein R represents an unsubstituted or substituted phenyl or lower alkyl group.

6. The process of claim 1 wherein the process variant (b) is carried out in a single reactor without isolating the condensate represented by formula VI and the derivative represented by formula VIII.

7. The process of claim 6 wherein in a single reactor, 4-biphenylylaldehyde of formula II and the

lactam compound of formula IV are heat-treated in the presence of a base, and then treated in an aqueous solvent.

8. A compound represented by the following formula VI

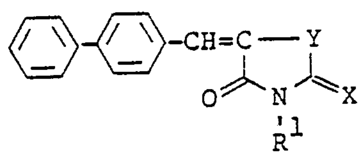

(VI)

wherein $R^1$ represents a hydrogen atom, a lower alkyl group or a carboxy-lower alkyl group, X represents an oxygen or sulfur atom, and Y represents a sulfur atom or —NH—.

## Patentansprüche

1. Verfahren zur Herstellung von 4-Biphenylylessigsäure oder ihres Salzes, dargestellt durch die folgende Formel I

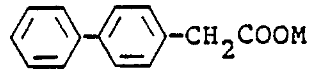

(I)

worin M ein Alkalimetall oder ein Wasserstoffatom bedeutet, dadurch gekennzeichnet, daß man entweder

(a) 4-Biphenylylaldehyd, dargestellt durch die folgende Formel II

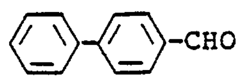

(II)

mit einem N-Acylglycin, dargestellt durch die folgende Formel III

$$RCO—NH—CH_2—COOH \quad (III)$$

worin R eine substituierte oder unsubstituierte Phenyl- oder Niedrigalkylgruppe bedeutet, in Anwesenheit einer Base umsetzt, um den 4-Biphenylylaldehyd in eine Azlactonverbindung, dargestellt durch die folgende Formel V

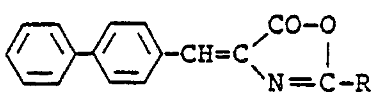

(V)

worin R die oben gegebene Definition besitzt, umzuwandeln, die entstehende Azlactonverbindung der Formel V erhitzt und sie gewünschtenfalls unter Bildung einer α-Ketosäure, dargestellt durch die folgende Formel VII

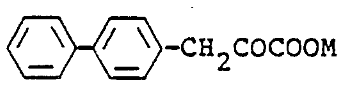

(VII)

worin M die oben gegebene Definition besitzt, neutralisiert und anschließend die α-Ketosäure decarboxyliert ; oder

(b) 4-Biphenylylaldehyd der folgenden Formel II

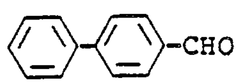

(II)

mit einer Lactamverbindung, dargestellt durch die folgende Formel IV

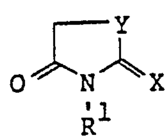

(IV)

worin $R^1$ ein Wasserstoffatom, eine Niedrigalkylgruppe oder eine Carboxy-Niedrigalkylgruppe bedeutet, X ein Sauerstoff- oder Schwefelatom bedeutet und Y ein Schwefelatom oder —NH— bedeutet, in Anwesenheit einer Base unter Bildung eines Kondensats, dargestellt durch die folgende Formel VI

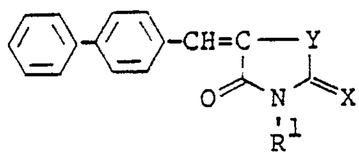

(VI)

worin $R^1$, X und Y die oben gegebenen Definitionen besitzen, umsetzt, das entstehende Kondensat der Formel VI in Anwesenheit einer Alkalibase erhitzt und es gewünschtenfalls unter Bildung eines Derivats, dargestellt durch die folgende Formel VIII

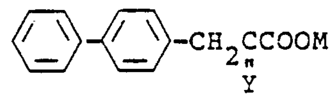

(VIII)

worin M und Y die oben gegebenen Definitionen besitzen, neutralisiert und das Derivat der Formel VIII mit einem Peroxid in Anwesenheit einer wäßrigen Base behandelt.

2. Verfahren nach Anspruch 1, gekennzeichnet, daß die Verfahrensvariante (a) in einem einzigen Reaktor durchgeführt wird, ohne daß die Azlactonverbindung der Formel V und die α-Ketoverbindung der Formel VII isoliert werden.

3. Verfahren nach Anspruch 2, dadurch gekennzeichnet, daß in einem einzigen Reaktor der 4-Biphenylylaldehyd der Formel II und das N-Acylglycin der Formel III in der Wärme in Anwesenheit einer Base behandelt werden und daß darauf die Decarboxylierung folgt.

4. Verfahren nach Anspruch 1, dadurch gekennzeichnet, daß die Decarboxylierung durch Behandlung mit einem Peroxid unter basischen Bedingungen durchgeführt wird.

5. Azlactonverbindung, dargestellt durch die folgende Formel V

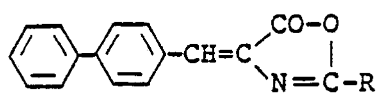

(V)

worin R eine unsubstituierte oder substituierte Phenyl- oder Niedrigalkylgruppe bedeutet.

6. Verfahren nach Anspruch 1, dadurch gekennzeichnet, daß die Verfahrensvariante (b) in einem einzigen Reaktor durchgeführt wird, ohne daß das Kondensat, welches durch die Formel VI, und das Derivat, welches durch die Formel VIII dargestellt werden, isoliert werden.

7. Verfahren nach Anspruch 6, dadurch gekennzeichnet, daß in einem einzigen Reaktor 4-Biphenylylaldehyd der Formel II und die Lactamverbindung der Formel IV in Anwesenheit einer Base in der Wärme behandelt werden und daß sie dann in einem wäßrigen Lösungsmittel behandelt werden.

8. Verbindung, dargestellt durch die folgende Formel VI

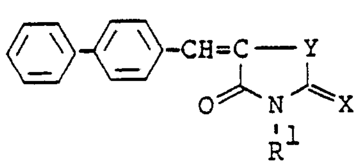

(VI)

worin $R^1$ ein Wasserstoffatom, eine Niedrigalkylgruppe oder eine Carboxy-Niedrigalkylgruppe bedeutet, X ein Sauerstoffoder Schwefelatom bedeutet und Y ein Schwefelatom oder —NH— bedeutet.

## Revendications

1. Procédé pour la production d'acide 4-biphénylylacétique ou d'un de ses sels représenté par la formule I suivante

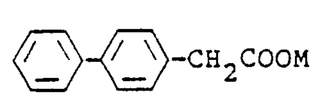

(I)

dans laquelle M représente un métal alcalin ou un atome d'hydrogène, qui consiste soit

(a) à faire réagir le 4-biphénylylaldéhyde représenté par la formule II suivante

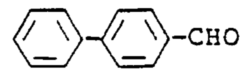

(II)

avec une N-acylglycine représentée par la formule III suivante

$$RCO—NH—CH_2COOH \qquad (III)$$

dans laquelle R représente un groupe phényle substitué ou non substitué ou un groupe alkyle inférieur, en présence d'une base pour convertir le 4-biphénylylaldéhyde en une azlactone représentée par la formule V suivante

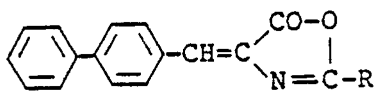

(V)

dans laquelle R est tel que défini ci-dessus, à chauffer l'azlactone résultante de formule V et, si on le désire, à la neutraliser pour former un alpha-céto-acide représenté par la formule VII suivante

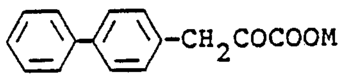

(VII)

dans laquelle M est tel que défini ci-dessus, et à décarboxyler ensuite l'alpha-céto-acide ; ou

(b) à faire réagir le 4-biphénylylaldéhyde de formule II suivante

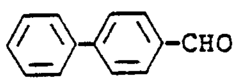

(II)

avec un lactame représenté par la formule IV suivante

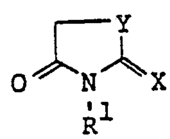

(IV)

dans laquelle $R^1$ représente un atome d'hydrogène, un groupe alkyle inférieur ou un groupe carboxy(alkyle inférieur), X représente un atome d'oxygène ou de soufre et Y représente un atome de soufre ou —NH—, en présence d'une base pour former un produit de condensation représenté par la formule VI suivante

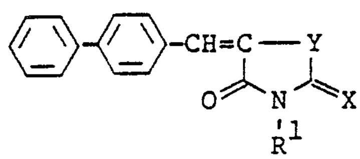

(VI)

dans laquelle $R^1$, X et Y sont tels que définis ci-dessus, à chauffer le produit de condensation résultant de formule VI en présence d'une base alcaline et, si on le désire, à le neutraliser pour former un dérivé représenté par la formule VIII suivante

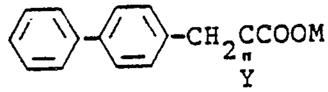

(VIII)

dans laquelle M et Y sont tels que définis ci-dessus, et à traiter le dérivé de formule VIII avec un peroxyde en présence d'une base aqueuse.

2. Procédé selon la revendication 1, dans lequel la variante de procédé (a) est exécutée dans un seul réacteur sans isolement de l'azlactone de formule V ni de l'alpha-céto-acide de formule VII.

3. Procédé selon la revendication 2, dans lequel, dans le même réacteur, le 4-biphénylylaldéhyde de formule II et la N-acylglycine de formule III sont traités thermiquement en présence d'une base, ce qui est suivi d'une décarboxylation.

4. Procédé selon la revendication 1, dans lequel la décarboxylation est effectuée par traitement avec un peroxyde dans des conditions basiques.

5. Azlactone qui est représentée par la formule V suivante

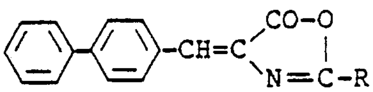

(V)

dans laquelle R représente un groupe alkyle inférieur ou phényle substitué ou non substitué.

6. Procédé selon la revendication 1, dans lequel la variante de procédé (b) est effectuée dans un seul réacteur sans isolement du produit de condensation représenté par la formule VI ni du dérivé représenté par la formule VIII.

7. Procédé selon la revendication 6, dans lequel, dans un seul réacteur, le 4-biphénylylaldéhyde de formule II et le lactame de formule IV sont traités thermiquement en présence d'une base, puis traités dans un solvant aqueux.

8. Composé représenté par la formule VI suivante

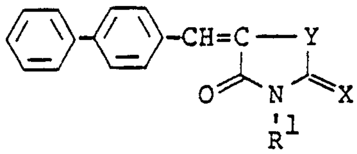

(VI)

dans laquelle $R^1$ représente un atome d'hydrogène, un groupe alkyle inférieur ou un groupe carboxy(alkyle inférieur), X représente un atome d'oxygène ou de soufre et Y représente un atome de soufre ou —NH—.